# EUROPEAN PATENT SPECIFICATION

(11) **EP 1 896 617 B1**
(45) Date of publication and mention of the grant of the patent: **02.01.2013**
(21) Application number: 06850497.6
(22) Date of filing: 31.05.2006
(51) Int. Cl.: C12Q 1/68, C12P 19/34

(54) **MULTIPLEX AMPLIFICATION OF SHORT NUCLEIC ACIDS**
MULTIPLEX-AMPLIFIKATION KURZER NUKLEINSÄUREN
AMPLIFICATION MULTIPLEXE D'ACIDES NUCLÉIQUES COURTS

(30) Priority: 31.05.2005 US 686521 P; 16.08.2005 US 708946 P; 24.08.2005 US 711480 P; 10.03.2006 US 781208 P; 07.04.2006 US 790472 P; 15.05.2006 US 800376 P
(43) Date of publication of application: 12.03.2008
(73) Proprietor: Life Technologies Corporation, Carlsbad, CA 92008 (US)
(72) Inventor: LAO, Kai Qin, Pleasanton, CA 94566 (US); LIVAK, Kenneth, J., San Jose, CA 95117 (US); STRAUS, Neil, A., Emeryville, CA 94608 (US)
(74) Representative: Grund, Martin
(86) International application number: PCT/US2006/021172
(87) International publication number: WO 2007/117256

(56) References cited:
- EP-A- 1 138 764
- WO-A-01/94634
- US-A1- 2004 014 058
- US-A1- 2004 203 061
- US-A1- 2005 074 788
- US-A1- 2005 266 418
- US-A1- 2005 270 275
- US-A1- 2006 057 595
- US-B1- 6 406 891
- US-B1- 6 548 250
- US-B1- 6 777 180
- CHEN ET AL: "Real-time PCR: advancing RNA interference and microRNA studies" PHARMACEUTICAL DISCOVERY, ADVANSTAR COMMUNICATIONS, INC., DULUTH, MN, US, 1 May 2005 (2005-05-01), XP002364909 ISSN: 1554-2068
- ELDERING ERIC ET AL: "Expression profiling via novel multiplex assay allows rapid assessment of gene regulation in defined signalling pathways." NUCLEIC ACIDS RESEARCH 1 DEC 2003, vol. 31, no. 23, 1 December 2003 (2003-12-01), page e153, XP002506295 ISSN: 1362-4962
- LIANG DER-CHERNG ET AL: "Multiplex RT-PCR assay for the detection of major fusion transcripts in Taiwanese children with B-lineage acute lymphoblastic leukemia" MEDICAL AND PEDIATRIC ONCOLOGY, vol. 39, no. 1, July 2002 (2002-07), pages 12-17, XP002506296 ISSN: 0098-1532
- TSE W T ET AL: "REVERSE TRANSCRIPTION AND DIRECT AMPLIFICATION OF CELLULAR RNA TRANSCRIPTS BY TAQ POLYMERASE" GENE, ELSEVIER, AMSTERDAM, NL, vol. 88, no. 2, 16 April 1990 (1990-04-16), pages 293-296, XP000226751 ISSN: 0378-1119
- LAO K ET AL: "Multiplexing RT-PCR for the detection of multiple miRNA species in small samples", BIOCHEMICAL AND BIOPHYSICAL RESEARCH COMMUNICATIONS, ACADEMIC PRESS INC. ORLANDO, FL, US LNKD- DOI:10.1016/J.BBRC.2006.02.106, vol. 343, no. 1, 28 February 2006 (2006-02-28), pages 85-89, XP024923539, ISSN: 0006-291X [retrieved on 2006-04-28]

## Description

### FIELD

The present teachings are in the field of molecular and cell biology, specifically in the field of multiplexed amplification of short nucleic acids such as micro RNAs.

### BACKGROUND

Numerous fields in molecular biology require the identification of target polynucleotide sequences. Reverse transcription and amplification are two frequently used procedures employed to query the identity of target polynucleotides. The increasing amount of sequence information available to scientists in the post-genomics era has produced an increased need for rapid, reliable, low-cost, high-throughput, sensitive, and accurate methods to query complex nucleic acid samples. Methods of defining and characterizing cells have been hindered by robust amplification technologies, as well as the molecular complexity of conventionally analyzed molecules such as messenger RNA. Micro RNAs are a recently discovered class of molecules that offer great promise in understanding cell function. However, quantitative analysis of micro RNA has been hindered by their relatively short size.

### SUMMARY

The invention is defined in the claims.

In one aspect, the invention is a method of amplifying a target micro RNA comprising; contacting the target micro RNA with a target-specific stem-loop reverse transcription primer, wherein the stem-loop reverse transcription primer comprises a 3 ' target-specific portion, a unique zip-coded stem comprising a unique zip-coded sequence that is associated with a particular micro RNA sequence, and a loop;
extending the stem-loop reverse transcription primer in a reverse transcription reaction to form a reverse transcription product;
performing a PCR-based pre-amplification on the reverse transcription product to form a PCR-based pre-amplification product, wherein the PCR-based pre-amplification comprises a forward primer and reverse primer, wherein the sequence of the reverse primer comprises substantially the same sequence as the loop of the at least one stem-loop reverse transcription primer, and a Tm-enhancing tail that is non-complementary to the reverse transcription product, and wherein the sequence of the forward primer comprises i) sequence that is complementary to the 3' end of the particular reverse transcription product sequence and ii) a 5' zip-code tail that is unique to the reverse transcription product sequence; and,
amplifying the target micro RNA.

In another aspect, the invention is a kit for amplifying at least 300 micro RNAs, said kit comprising; at least 300 stem-loop reverse transcription primers, wherein each of the stem-loop reverse transcription primer comprises a 3'target-specific portion, a unique zip-coded stem comprising a unique zip-coded sequence that is associated with a particular micro RNA sequence, and a loop and wherein each of the at least 300 stem-loop reverse transcription primers contains substantially the same loop sequence; at least 300 forward primers, wherein each forward primer comprises a distinct 3' target- specific portion and a distinct 5' tail that is unique to the reverse transcription product sequence ; a universal reverse primer, wherein the sequence of the universal reverse primer comprises substantially the same sequence as the sequence contained in the loop of the at least 300 stem-loop reverse transcription primers, and, a Tm-enhancing tail that is non-complementary to the reverse transcription product.

In some embodiments, the present teachings provide a method of quantitating at least 300 different short target nucleic acids, wherein each short target nucleic acid is 18-30 nucleotides in length, said method comprising; contacting the at least 300 different short target nucleic acids with at least 300 different target-specific stem-loop reverse transcription primers, wherein each of the at least 300 stem-loop reverse transcription primers comprises a unique 3' target-specific portion, a unique zip-coded stem, and a loop; extending the at least 300 stem-loop reverse transcription primers in a reverse transcription reaction to form a collection of reverse transcription products; performing a PCR-based pre-amplification on the collection of reverse transcription products to form a collection of PCR-based pre-amplification products, wherein the PCR-based pre-amplification comprises at least 300 different forward primers and at least one reverse primer, wherein the sequence of the reverse primer comprises substantially the same sequence as the loop of the at least one stem-loop reverse transcription primer, and a Tm-enhancing tail; wherein each of the at least 300 different forward primers comprises i) a 3' target-specific portion that is complementary to the 5' end of a particular reverse transcription product sequence and ii) a 5' zip-code tail that is unique to a particular reverse transcription product sequence; and, dividing the collection of PCR-based pre-amplification products into at least 300 different reaction vessels; performing a decoding PCR in each of the at least 300 different reaction vessels, wherein each decoding PCR comprises a forward primer that comprises substantially the same sequence as the forward primer in the PCR-based pre-amplification reaction for a particular target nucleic acid sequence, a reverse primer, and, a detector probe, wherein the sequence of the detector probe comprises i) a sequence of at least 6 nucleobases that is the same as the 3' stem region of a particular stem-loop reverse transcription primer, and, ii) a sequence of at least 6 nucleobases that is complementary to the short target nucleic acid queried by the particular stem-loop reverse transcription primer; detecting the detector probe in each of the at least 300 different reaction vessels; and, quantifying the at least 300 different target short nucleic acids.

### BRIEF DESCRIPTION OF THE DRAWINGS

The skilled artisan will understand that the drawings, described below, are for illustration purposes only. The drawings are not intended to limit the scope of the present teachings in any way.
Figure 1 depicts certain aspects of various compositions according to some embodiments of the present teachings
Figure 2 depicts one workflow according to some embodiments of the present teachings.
Figure 3 depicts certain aspects of various compositions according to some embodiments of the present teachings.
Figure 4 depicts certain aspects of various compositions according to some embodiments of the present teachings.
Figure 5 depicts certain aspects of various compositions according to some embodiments of the present teachings.
Figure 6 depicts illustrative date in the form of Ct values according to some embodiments of the present teachings.

### DESCRIPTION OF EXEMPLARY EMBODIMENTS

Aspects of the present teachings may be further understood in light of the following examples, which should not be construed as limiting the scope of the present teachings in any way. The section headings used herein are for organizational purposes only and are not to be construed as limiting the described subject matter in any way. When definitions of terms in references appear to differ from the definitions provided in the present teachings, the definition provided in the present teachings shall control. It will be appreciated that there is an implied "about" prior to the temperatures, concentrations, times, etc discussed in the present teachings, such that slight and insubstantial deviations are within the scope of the present teachings herein. In this application, the use of the singular includes the plural unless specifically stated otherwise. For example, "a primer" means that more than one primer can, but need not, be present; for example but without limitation, one or more copies of a particular primer species, as well as one or more versions of a particular primer type, for example but not limited to, a multiplicity of different forward primers. Also, the use of "comprise", "comprises", "comprising", "contain", "contains", "containing", "include", "includes", and "including" are not intended to be limiting. It is to be understood that both the foregoing general description and the following detailed description are exemplary and explanatory only and are not restrictive of the invention.

### Some Definitions

As used herein, the term "target nucleic acid" refers to a polynucleotide sequence that is sought to be amplified and/or quantified. The target polynucleotide can be obtained from any source, and can comprise any number of different compositional components. For example, the target can be nucleic acid (e.g. DNA or RNA), transfer RNA, siRNA, and can comprise nucleic acid analogs or other nucleic acid mimic, though typically the target will be messenger RNA (mRNA) and/or micro RNA (miRNA). The target can be methylated, non-methylated, or both. The target can be bisulfite-treated and non-methylated cytosines converted to uracil. Further, it will be appreciated that "target polynucleotide" can refer to the target polynucleotide itself, as well as surrogates thereof, for example amplification products, and native sequences. In some embodiments, the target polynucleotide is a short DNA molecule derived from a degraded source, such as can be found in for example but not limited to forensics samples (see for example Butler, 2001, Forensic DNA Typing: Biology and Technology Behind STR Markers. The target polynucleotides of the present teachings can be derived from any of a number of sources, including without limitation, viruses, prokaryotes, eukaryotes, for example but not limited to plants, fungi, and animals. These sources may include, but are not limited to, whole blood, a tissue biopsy, lymph, bone marrow, amniotic fluid, hair, skin, semen, biowarfare agents, anal secretions, vaginal secretions, perspiration, saliva, buccal swabs, various environmental samples (for example, agricultural, water, and soil), research samples generally, purified samples generally, cultured cells, and lysed cells. It will be appreciated that target polynucleotides can be isolated from samples using any of a variety of procedures known in the art, for example the Applied Biosystems ABI Prism ^{™} 6100 Nucleic Acid PrepStation, and the ABI Prism ^{™} 6700 Automated Nucleic Acid Workstation, Boom et al., U.S. Patent 5,234,809., mirVana RNA isolation kit (Ambion), etc. It will be appreciated that target polynucleotides can be cut or sheared prior to analysis, including the use of such procedures as mechanical force, sonication, restriction endonuclease cleavage, or any method known in the art. In general, the target polynucleotides of the present teachings will be single stranded, though in some embodiments the target polynucleotide can be double stranded, and a single strand can result from denaturation.

As used herein, the term "reverse transcription reaction" refers to an elongation reaction in which the 3' target-specific portion of a stem-loop primer is extended to form an extension reaction product comprising a strand complementary to the target polynucleotide. In some embodiments, the target polynucleotide is a miRNA molecule and the extension reaction is a reverse transcription reaction comprising a reverse transcriptase, where the 3' end of a stem-loop primer is extended. In some embodiments, the extension reaction is a reverse transcription reaction comprising a polymerase derived from a Eubacteria. In some embodiments, the extension reaction can comprise rTth polymerase, for example as commercially available from Applied Biosystems catalog number N808-0192, and N808-0098. In some embodiments, the target polynucleotide is a miRNA or other RNA molecule, and the use of polymerases that also comprise reverse transcription properties can allow for a first reverse transcription reaction followed thereafter by an amplification reaction such as a multi-plexed PCR-based pre-amplification in the same reaction vessel, thereby allowing for the consolidation of two reactions in single reaction vessel. In some embodiments, the target polynucleotide is a DNA molecule and the extension reaction comprises a polymerase and results in the synthesis of a complementary strand of DNA. The term reverse transcription also includes also includes the synthesis of a DNA complement of a template DNA molecule. Similarly, a reverse transcription product can be a DNA molecule synthesized in a reverse transcription reaction, which is thus complementary to the template.

As used herein, the term "reverse primer" refers to a primer that when extended in a reaction such as a reverse transcription reaction forms a complementary strand. Following the extension reaction, a forward primer can hybridize to the synthesized strand and be extended. In some embodiments, a stem-loop reverse transcription primer functions as a first reverse primer in a reverse transcription reaction. Thereafter, a second reverse primer that was encoded by the stem-loop primer can be employed, and the second reverse primer can hybridize to the strand resulting from extension of the forward primer. In those embodiments in which a large number of the stem-loop reverse transcription primers contain the same, or substantially the same, sequence in their loop, the reverse primer used in the PCR-based pre-amplification reaction is referred to as a universal reverse primer. This universal reverse primer will generally comprise the sequence of the loop, as well as a Tm-enhancing tail.

As used herein, the term "hybridization" refers to the complementary base-pairing interaction of one nucleic acid with another nucleic acid that results in the formation of a duplex, triplex, or other higher-ordered structure, and is used herein interchangeably with "annealing." Typically, the primary interaction is base specific, e.g., A/T and G/C, by Watson/Crick and Hoogsteen-type hydrogen bonding. Base-stacking and hydrophobic interactions can also contribute to duplex stability. Conditions for hybridizing primers to complementary and substantially complementary target sequences are well known, e.g., as described in Nucleic Acid Hybridization, A Practical Approach, B. Hames and S. Higgins, eds., IRL Press, Washington, D.C. (1985) and J. Wetmur and N. Davidson, Mol. Biol. 31:349 et seq. (1968). In general, whether such annealing takes place is influenced by, among other things, the length of the polynucleotides and the complementary, the pH, the temperature, the presence of mono- and divalent cations, the proportion of G and C nucleotides in the hybridizing region, the viscosity of the medium, and the presence of denaturants. Such variables influence the time required for hybridization. Thus, the preferred annealing conditions will depend upon the particular application. Such conditions, however, can be routinely determined by the person of ordinary skill in the art without undue experimentation. It will be appreciated that complementarity need not be perfect; there can be a small number of base pair mismatches that will minimally interfere with hybridization between the target sequence and the single stranded nucleic acids of the present teachings. However, if the number of base pair mismatches is so great that no hybridization can occur under minimally stringent conditions then the sequence is generally not a complementary target sequence. Thus, complementarity herein is meant that primers are sufficiently complementary to the target sequence to hybridize under the selected reaction conditions to achieve the ends of the present teachings.

As used herein, the term "amplifying" refers to any means by which at least a part of a target polynucleotide and/or target polynucleotide surrogate is reproduced, typically in a template-dependent manner, including without limitation, a broad range of techniques for amplifying nucleic acid sequences, either linearly or exponentially. In some embodiments, amplification can be achieved in a self-contained integrated approach comprising sample preparation and detection, as described for example in U.S. Patent Nos. 6,153,425 and 6,649,378. Amplifying nucleic acids can employ reversibly modified enzymes, for example but not limited to those described in U.S. Patent No. 5,773,258. The present teachings also contemplate various uracil-based decontamination strategies, wherein for example uracil can be incorporated into an amplification reaction, and subsequent carry-over products removed with various glycosylase treatments (see for example U.S. Patent No. 5,536,649, and U.S. Non-Provisional Patent Application 11/173,112 to Andersen et al.,). Those in the art will understand that any protein with the desired enzymatic activity can be used in the disclosed methods and kits. Descriptions of DNA polymerases, including reverse transcriptases, uracil N-glycosylase, and the like, can be found in, among other places, Twyman, Advanced Molecular Biology, BIOS Scientific Publishers, 1999; Enzyme Resource Guide, rev. 092298, Promega, 1998; Sambrook and Russell; Sambrook et al.; Lehninger; PCR: The Basics; and Ausbel et al.

The term "corresponding" as used herein refers to a specific relationship between the elements to which the term refers. Some non-limiting examples of corresponding include: a reverse primer can correspond with a target nucleic acid, and vice versa. A forward primer can correspond with a target nucleic acid, and vice versa.

As used herein, the term "reaction vessel" generally refers to any container in which a reaction can occur in accordance with the present teachings. In some embodiments, a reaction vessel can be an eppendorf tube, and other containers of the sort in common practice in modem molecular biology laboratories. In some embodiments, a reaction vessel can be a well in microtitre plate, a spot on a glass slide, or a well in an Applied Biosystems TaqMan Low Density Array for gene expression (formerly MicroCard ^{™}). For example, a plurality of reaction vessels can reside on the same support. In some embodiments, lab-on-a-chip like devices, available for example from Caliper and Fluidigm, can provide for reaction vessels. In some embodiments, various microfluidic approaches as described in U.S. Non-Provisional Patent Application 11/059,824 to Wenz et al., can be employed. It will be recognized that a variety of reaction vessels are available in the art and can be used in the context of the present teachings.

As used herein, the term "PCR-based pre-amplification" refers to a process wherein a plurality of primer pairs are included in a multiplexed PCR amplification reaction, and the multiplexed amplification reaction undergoes a limited number of cycles so that the PCR-based pre-amplification reaction ends prior to the PCR plateau and/or reagent depletion. The term "PCR-based pre-amplification" can be considered to indicate that a secondary amplification reaction is subsequently performed, typically of lower plexy level than the PCR-based pre-amplification reaction. This secondary amplification reaction, typically a plurality of separate secondary amplification reactions, can employ primer pairs encoded by the primers used in the multiplexed PCR-based pre-amplification reaction. However, each secondary amplification reaction typically comprises a single or a few primer pairs. Further examples of PCR-based pre-amplification approaches can be found for example in U.S. Patent 6,605,451 to Xtrana, and U.S. Patent Application 10/723,520 to Andersen et al.,

As used herein, the term "detection" refers to any of a variety of ways of determining the presence and/or quantity and/or identity of a target polynucleotide. In some embodiments employing a donor moiety and signal moiety, one may use certain energy-transfer fluorescent dyes. Certain nonlimiting exemplary pairs of donors (donor moieties) and acceptors (signal moieties) are illustrated, e.g., in U.S. Patent Nos. 5,863,727; 5,800,996; and 5,945,526. Use of some combinations of a donor and an acceptor have been called FRET (Fluorescent Resonance Energy Transfer). In some embodiments, fluorophores that can be used as signaling probes include, but are not limited to, rhodamine, cyanine 3 (Cy 3), cyanine 5 (Cy 5), fluorescein, Vic™, Liz™, Tamra™, 5-Fam™, 6-Fam™, and Texas Red (Molecular Probes). (Vic™, Liz™, Tamra™, 5-Fam™, and 6-Fam™ (all available from Applied Biosystems, Foster City, CA.). In some embodiments, the amount of detector probe that gives a fluorescent signal in response to an excited light typically relates to the amount of nucleic acid produced in the amplification reaction. Thus, in some embodiments, the amount of fluorescent signal is related to the amount of product created in the amplification reaction. In such embodiments, one can therefore measure the amount of amplification product by measuring the intensity of the fluorescent signal from the fluorescent indicator. According to some embodiments, one can employ an internal standard to quantify the amplification product indicated by the fluorescent signal. See, e.g., U.S. Patent No. 5,736,333. Devices have been developed that can perform a thermal cycling reaction with compositions containing a fluorescent indicator, emit a light beam of a specified wavelength, read the intensity of the fluorescent dye, and display the intensity of fluorescence after each cycle. Devices comprising a thermal cycler, light beam emitter, and a fluorescent signal detector, have been described, e.g., in U.S. Patent Nos. 5,928,907; 6,015,674; and 6,174,670, and include, but are not limited to the ABI Prism® 7700 Sequence Detection System (Applied Biosystems, Foster City, California), the ABI GeneAmp® 5700 Sequence Detection System (Applied Biosystems, Foster City, California), the ABI GeneAmp® 7300 Sequence Detection System (Applied Biosystems, Foster City, California), and the ABI GeneAmp® 7500 Sequence Detection System (Applied Biosystems). In some embodiments, each of these functions can be performed by separate devices. For example, if one employs a Q-beta replicase reaction for amplification, the reaction may not take place in a thermal cycler, but could include a light beam emitted at a specific wavelength, detection of the fluorescent signal, and calculation and display of the amount of amplification product. In some embodiments, combined thermal cycling and fluorescence detecting devices can be used for precise quantification of target nucleic acid sequences in samples. In some embodiments, fluorescent signals can be detected and displayed during and/or after one or more thermal cycles, thus permitting monitoring of amplification products as the reactions occur in "real time." In some embodiments, one can use the amount of amplification product and number of amplification cycles to calculate how much of the target nucleic acid sequence was in the sample prior to amplification. In some embodiments, one could simply monitor the amount of amplification product after a predetermined number of cycles sufficient to indicate the presence of the target nucleic acid sequence in the sample. One skilled in the art can easily determine, for any given sample type, primer sequence, and reaction condition, how many cycles are sufficient to determine the presence of a given target polynucleotide. As used herein, determining the presence of a target can comprise identifying it, as well as optionally quantifying it. In some embodiments, the amplification products can be scored as positive or negative as soon as a given number of cycles is complete. In some embodiments, the results may be transmitted electronically directly to a database and tabulated. Thus, in some embodiments, large numbers of samples can be processed and analyzed with less time and labor when such an instrument is used. In some embodiments, different detector probes may distinguish between different target polynucleotides. A non-limiting example of such a probe is a 5'-nuclease fluorescent probe, such as a TaqMan® probe molecule, wherein a fluorescent molecule is attached to a fluorescence-quenching molecule through an oligonucleotide link element. In some embodiments, the oligonucleotide link element of the 5'-nuclease fluorescent probe binds to a specific sequence of an identifying portion or its complement. In some embodiments, different 5'-nuclease fluorescent probes, each fluorescing at different wavelengths, can distinguish between different amplification products within the same amplification reaction. For example, in some embodiments, one could use two different 5'-nuclease fluorescent probes that fluoresce at two different wavelengths (WL_{A} and WL_{B}) and that are specific to two different regions of two different extension reaction products (A' and B', respectively). Amplification product A' is formed if target polynucleotide A is in the sample, and amplification product B' is formed if target polynucleotide B is in the sample. In some embodiments, amplification product A' and/or B' may form even if the appropriate target polynucleotide is not in the sample, but such occurs to a measurably lesser extent than when the appropriate target polynucleotide is in the sample. After amplification, one can determine which specific target nucleic acid sequences are present in the sample based on the wavelength of signal detected and their intensity. Thus, if an appropriate detectable signal value of only wavelength WL_{A} is detected, one would know that the sample includes target polynucleotide A, but not target polynucleotide B. If an appropriate detectable signal value of both wavelengths WL_{A} and WL_{B} are detected, one would know that the sample includes both target polynucleotide A and target polynucleotide B. In some embodiments, detection can be achieved by various microarrays and related software such as the Applied Biosystems Array System with the Applied Biosystems 1700 Chemiluminescent Microarray Analyzer and other commercially available array systems available from Affymetrix, Agilent, Illumina, and Amersham Biosciences, among others (*see also* Gerry et al., J. Mol. Biol. 292:251-62, 1999; De Bellis et al., Minerva Biotec 14:247-52, 2002; and Stears et al., Nat. Med. 9:140-45, including supplements, 2003). It will also be appreciated that detection can comprise reporter groups that are incorporated into the reaction products, either as part of labeled primers or due to the incorporation of labeled dNTPs during an amplification, or attached to reaction products, for example but not limited to, via hybridization tag complements comprising reporter groups or via linker arms that are integral or attached to reaction products. Detection of unlabeled reaction products, for example using mass spectrometry, is also within the scope of the current teachings.

As used herein, the term "detector probe" refers to a molecule used in an amplification reaction, typically for quantitative or real-time PCR analysis, as well as end-point analysis. Such detector probes can be used to monitor the amplification of the target micro RNA and/or control nucleic acids such as endogenous control small nucleic acids and/or synthetic internal controls. In some embodiments, detector probes present in an amplification reaction are suitable for monitoring the amount of amplicon(s) produced as a function of time. Such detector probes include, but are not limited to, the 5'-exonuclease assay (TaqMan ® probes described herein (see also U.S. Patent No. 5,538,848) various stem-loop molecular beacons (see e.g., U.S. Patent Nos. 6,103,476 and 5,925,517 and Tyagi and Kramer, 1996, Nature Biotechnology 14:303-308), stemless or linear beacons (see, e.g., WO 99/21881), PNA Molecular Beacons ™ (see, e.g., U.S. Patent Nos. 6,355,421 and 6,593,091), linear PNA beacons (see, e.g., Kubista et al., 2001, SPIE 4264:53-58), non-FRET probes (see, e.g., U.S. Patent No. 6,150,097), Sunrise ®/Amplifluor ® probes (U.S. Patent No. 6,548,250), stem-loop and duplex Scorpion ™ probes (Solinas et al., 2001, Nucleic Acids Research 29:E96 and U.S. Patent No. 6,589,743), bulge loop probes (U.S. Patent No. 6,590,091), pseudo knot probes (U.S. Patent No. 6,589,250), cyclicons (U.S. Patent No. 6,383,752), MGB Eclipse ™ probe (Epoch Biosciences), hairpin probes (U.S. Patent No. 6,596,490), peptide nucleic acid (PNA) light-up probes, self-assembled nanoparticle probes, and ferrocene-modified probes described, for example, in U.S. Patent No. 6,485,901; Mhlanga et al., 2001, Methods 25:463-471; Whitcombe et al., 1999, Nature Biotechnology. 17:804-807; Isacsson et al., 2000, Molecular Cell Probes. 14:321-328; Svanvik et al., 2000, Anal Biochem. 281:26-35; Wolffs et al., 2001, Biotechniques 766:769-771; Tsourkas et al., 2002, Nucleic Acids Research. 30:4208-4215; Riccelli et al., 2002, Nucleic Acids Research 30:4088-4093; Zhang et al., 2002 Shanghai. 34:329-332; Maxwell et al., 2002, J. Am. Chem. Soc. 124:9606-9612; Broude et al., 2002, Trends Biotechnol. 20:249-56; Huang et al., 2002, Chem Res. Toxicol. 15:118-126; and Yu et al., 2001, J. Am. Chem. Soc 14:11155-11161. Detector probes can also comprise quenchers, including without limitation black hole quenchers (Biosearch), Iowa Black (IDT), QSY quencher (Molecular Probes), and Dabsyl and Dabcel sulfonate/carboxylate Quenchers (Epoch). Detector probes can also comprise two probes, wherein for example a fluor is on one probe, and a quencher is on the other probe, wherein hybridization of the two probes together on a target quenches the signal, or wherein hybridization on the target alters the signal signature via a change in fluorescence. Illustrative detector probes comprising two probes wherein one molecule is an L-DNA and the other molecule is a PNA can be found in U.S. Non-Provisional Patent Application 11/172,280 to Lao et al., Detector probes can also comprise sulfonate derivatives of fluorescenin dyes with SO3 instead of the carboxylate group, phosphoramidite forms of fluorescein, phosphoramidite forms of CY 5 (commercially available for example from Amersham). In some embodiments, intercalating labels are used such as ethidium bromide, SYBR® Green I (Molecular Probes), and PicoGreen® (Molecular Probes), thereby allowing visualization in real-time, or end point, of an amplification product in the absence of a detector probe. In some embodiments, real-time visualization can comprise both an intercalating detector probe and a sequence-based detector probe can be employed. In some embodiments, the detector probe is at least partially quenched when not hybridized to a complementary sequence in the amplification reaction, and is at least partially unquenched when hybridized to a complementary sequence in the amplification reaction. In some embodiments, probes can further comprise various modifications such as a minor groove binder (see for example U.S. Patent 6,486,308) to further provide desirable thermodynamic characteristics. In some embodiments, detector probes can correspond to the zip-code introduced by the stem-loop reverse transcription primer.

In some embodiments, the detector probe comprises i) a sequence of at least 6 nucleobases that is the same as the 3' stem region of the stem-loop reverse primer, and, ii) a sequence of at least 6 nucleobases that is complementary to the target nucleic acid. In some embodiments, the detector probe comprises i) a sequence of at least 7 nucleobases that is the same as the 3' stem region of the stem-loop reverse primer, and, ii) a sequence of at least 7 nucleobases that is complementary to the target nucleic acid. In some embodiments, the detector probe comprises i) a sequence of at least 8 nucleobases that is the same as the 3' stem region of the stem-loop reverse primer, and, ii) a sequence of at least 8 nucleobases that is complementary to the target nucleic acid. Of course, lengths greater than 8 can be employed. Generally, lengths less than 5 nucleobases will be discouraged since such introduce non-specific interactions. It will also be appreciated that detector probe can comprise the sequence of at least X nucleobases that is the same as the 3' stem region of the stem-loop reverse primer, and, ii) a sequence of at least Y nucleobases that is complementary to the target nucleic acid, wherein X and Y are different numbers.

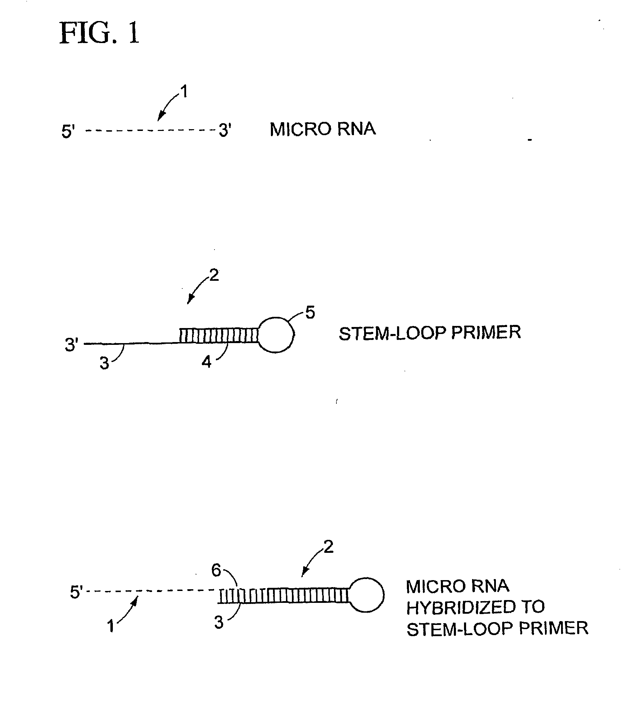

As used herein, the term "stem-loop primer" refers to a molecule comprising a 3' target specific portion, a stem, and a loop. Illustrative stem-loop primers are depicted in Figure 1, elsewhere in the present teachings, and in U.S. Patent Application 10/947,460 to Chen et al., The term "3' target-specific portion" refers to the single stranded portion of a stem-loop primer that is complementary to a target polynucleotide such as target micro RNA or endogenous control small RNA. The 3' target-specific portion is located downstream from the stem of the stem-loop primer. Generally, the 3' target-specific portion is between 6 and 9 nucleotides long. In some embodiments, the 3' target-specific portion is 7 nucleotides long. It will be appreciated that routine experimentation can produce other lengths, and that 3' target-specific portions that are longer than 8 nucleotides or shorter than 6 nucleotides are also contemplated by the present teachings. Generally, the 3'-most nucleotides of the 3' target-specific portion should have minimal complementarity overlap, or no overlap at all, with the 3' nucleotides of the forward primer; it will be appreciated that overlap in these regions can produce undesired primer dimer amplification products in subsequent amplification reactions. In some embodiments, the overlap between the 3'-most nucleotides of the 3' target-specific portion and the 3' nucleotides of the forward primer is 0, 1, 2, or 3 nucleotides. In some embodiments, greater than 3 nucleotides can be complementary between the 3'-most nucleotides of the 3' target-specific portion and the 3' nucleotides of the forward primer, but generally such scenarios will be accompanied by additional non-complementary nucleotides interspersed therein. In some embodiments, modified bases such as LNA can be used in the 3' target specific portion to increase the Tm of the stem-loop primer (see for example Petersen et al., Trends in Biochemistry (2003), 21:2:74-81). In some embodiments, universal bases can be used, for example to allow for smaller libraries of stem-loop primers. In some embodiments, modifications including but not limited to LNAs and universal bases can improve reverse transcription specificity and potentially enhance detection specificity. The term "stem" refers to the double stranded region of the stem-loop primer that is between the 3' target-specific portion and the loop, and is discussed more fully below. The term "loop" refers to a region of the stem-loop primer that is located between the two complementary strands of the stem, as depicted for example in Figure 1. Typically, the loop comprises single stranded nucleotides, though other moieties including modified DNA or RNA, Carbon spacers such as C18, and/or PEG (polyethylene glycol) are also possible. Generally, the loop is between 4 and 30 nucleotides long. In some embodiments, the loop is between 14 and 18 nucleotides long. In some embodiments, the loop is 16 nucleotides long. Those in the art will appreciate that loops shorter that 4 nucleotides and longer than 20 nucleotides can be identified in the course of routine methodology and without undue experimentation, and that such shorter and longer loops are contemplated by the present teachings. In some embodiments, the loop can comprise an identifying portion, also known as a "zip-code."

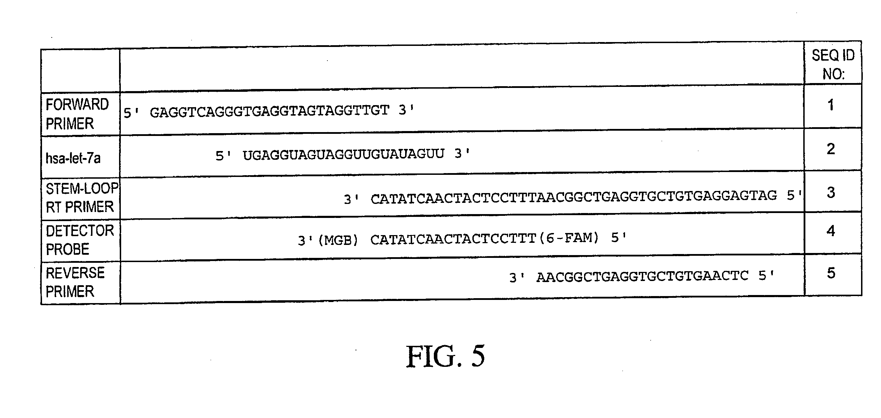

As used herein, the term "comprises at least 70 percent of the sequence of the loop" refers to the sequence of the reverse primer relative to the sequence of the loop of the stem-loop reverse transcription primer. For example, in reference to Figure 5, and the sequences contained therein, the stem-loop reverse transcription primer is
SEQ ID NO:3 3'CATATCAACTACTCCT**TTAACGGCTGAGGTGCTGTG**AGGAGTAG5'

The stem is underlined. The loop is bold. The reverse primer sequence is
SEQ ID NO:5 3'AACGGCTGAGGTGCTGTGAACTC5'

Thus, the reverse primer sequence is two nucleobases shorter than the loop. Hence, since the loop is 20 nucleobases, and the reverse primer is 18 nucleobases, it can be said that the reverse primer comprises 18/20, or, 90 percent, of the sequence of the loop. It is in this sense that the expression "comprises at least 70 percent of the sequence of the loop" is used in the present teachings.

Of course, the present teachings further contemplate embodiments in which the would-be copy-cat would attempt to merely change a base or two, or three, in the sequence of the reverse primer relative to the loop. The present teachings contemplate these and analogous scenarios. Hence, when the phrase "substantially the same as" is used to refer to a sequence, it will be appreciated that this is intended to include within the scope of the present teaching slight deviations in the sequences employed. Such slight alterations are clearly contemplated by the present teachings. For the avoidance of doubt, "substantially the same as" will typically mean that the sequence is at least 90 percent the same as the corresponding sequence. Further, when referring for example to the fact that the reverse primer comprises substantially the same sequence as the sequence contained in the loop of the stem-loop reverse transcription primer, it will be appreciated that "at least 70 percent of the sequence of the loop" applies and, further that of that at least 70 percent, at least 90 percent is the same as the corresponding loop sequence.

In further reference to the illustrative sequences presented in Figure 5, as used herein, the term "zip-coded stem" refers to the double-stranded region of the stem-loop reverse transcription primer. The stem of the stem-loop reverse transcription primer in Figure 5 is 8 base-pairs in length. In some embodiments, the stem can be 9 base-pairs in length. In some embodiments, the stem can be 10 base-pairs in length. In some embodiments, the stem can be 11 base-pairs in length. In some embodiments, the stem can be 12 base-pairs in length. In some embodiments, the stem can be 13 base-pairs in length. In some embodiments, the stem can be 14 base-pairs in length. Generally, longer stems are possible, but will come at the cost of increased expense in oligonucleotide manufacturing, and will further add to reaction complexity. In some embodiments, the stem can be 7 base-pairs in length. In some embodiments, the stem can be 6 base-pairs in length. Stems shorter than 6 base-pairs in length are possible, but are done at the sacrifice of specificity at the level of detector probe binding.

As used herein, the term "3' stem region of the stem-loop reverse transcription primer" refers to one of the strands of the stem, in particular the strand nearest to the 3' end of the stem-loop reverse transcription primer. The other stand can be referred to as the "5' stem region of the stem-loop reverse transcription primer."

As used herein, the term "Tm-enhancing tail" refers to a small number of nucleobases, typically between 3 and 10, that are included at the 5' end of the reverse primer used in the PCR-based pre-amplification reaction. The Tm-enhancing tail is not complementary to the reverse transcription product. In some embodiments, the Tm-enhancing tail is 4 bases. In some embodiments, the Tm-enhancing tail is 5 bases. In some embodiments, the Tm-enhancing tail is 6 bases. In some embodiments, the Tm-enhancing tail is 7 bases. Generally, longer Tm enhancing tails are possible, but will come at the cost of increased expense in oligonucleotide manufacturing, will further add to reaction complexity, and may raise the Tm to undesirable levels.

As used herein, the term "5' zip-code tail" refers to a sequence of nucleobases, typically between 7 and 15, that are included at the 5' end of the forward primer used in the PCR-based pre-amplification reaction. The 5' zip-code tail is associated with a particular target nucleic acid sequence. That is to say, a target nucleic acid such as the micro RNA let-7a can be amplified in a PCR-based pre-amplification reaction, wherein the 5' zip-code tail of the forward primer is uniquely associated with let-7a. This is achieved by the judicious note-keeping of which zip-code is paired with which 3' target-specific portion in a forward primer, thus allowing for the zip-code to vary along with a given target nucleic acid. Therefore, the 5' zip-code tail of a forward primer querying, for example, the micro RNA miR-297, will be different from the 5' zip-code tail of the forward primer querying the micro RNA let-7a. This encoding allows for greater performance in highly multiplexed environments, and allows for a large number of target nucleic acids of interest to be uniquely encoded with zip-code information by the 5' zip-code tail of a corresponding forward primer. Thus, in some phrasings, the present teachings will refer to a 5' zip-code tail that is unique to a particular reverse transcription product sequence. In some embodiments, the zip-code tail is 8 nucleobases. In some embodiments, the zip-code tail is 9 nucleobases. In some embodiments, the zip-code tail is 10 nucleobases. In some embodiments, the zip-code tail is 11 nucleobases. In some embodiments, the zip-code tail is 12 nucleobases. Generally, longer stems are 5' zip-code tails are possible, but will come at the cost of increased expense in oligo manufacturing, and will further add to reaction complexity. Descriptions of zip-codes can be found in, among other places, U.S. Patent Nos. 6,309,829 (referred to as "tag segment" therein); 6,451,525 (referred to as "tag segment" therein); 6,309,829 (referred to as "tag segment" therein); 5,981,176 (referred to as "grid oligonucleotides" therein); 5,935,793 (referred to as "identifier tags" therein); and PCT Publication No. WO 01/92579 (referred to as "addressable support-specific sequences" therein).

### Exemplary Embodiments

Figure 1 depicts certain compositions according to some embodiments of the present teachings. Top, a miRNA molecule (1, dashed line) is depicted. Middle, a stem-loop reverse transcription primer (2) is depicted, illustrating a 3' target-specific portion (3), a stem (4), and a loop (5). Bottom, the miRNA (1) hybridized to the stem-loop primer (2) is depicted, illustrating the 3' target-specific portion (3) of the stem-loop primer (2) hybridized to the 3' end region (6) of the miRNA (1).

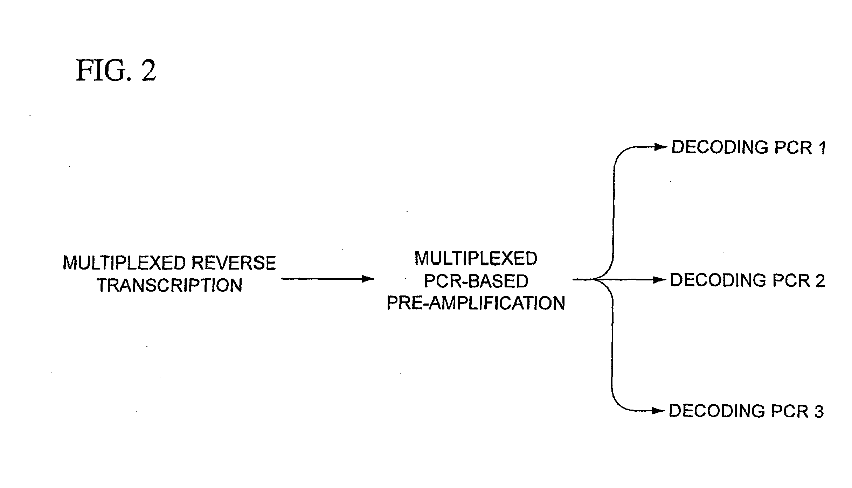

Figure 2 illustrates an overview of a process according to some embodiments of the present teachings. Here, a multiplexed reverse transcription reaction is performed on a plurality of nucleic acids, such as micro RNAs. This reverse transcription can be cycled according to some embodiments of the present teachings, for example in a two segment cycling procedure, or a three segment cycling procedure. Also, this reverse transcription reaction can comprise stem-loop reverse transcription primers. Following the reverse transcription reaction, and optionally occurring in the same vessel as the reverse transcription reaction, a multiplexed PCR-based pre-amplification can be performed This multiplexed PCR-based pre-amplification can then be followed by a plurality of separate decoding PCRs, here a PCR 1, a PCR 2, and a PCR 3. PCR-based pre-amplification of target polynucleotides (see for example U.S. Patent Application 10/723,520 to Andersen et al., and U.S. Patent 6,605,451 to Xtrana) can pre-amplify cDNA more than 1,000-fold for up to kilo-plex numbers of target nucleic acids. Subsequent lower-plex amplification reactions can then decode these multiplexed reactions, thereby allowing for detection and quantitation of a plurality of different target polynucleotides with an economy of reagents. The identify and quantity of one, some, or all, of the micro RNAs in a given cell type, or collection of cell types, can be referred to as a 'micro RNA signature', and can be achieved by application of the methods of the present teachings. Discussion of signatures can be found, for example, in U.S. Patent 6,110,711 and U.S. Patent 5,514,545, wherein messenger RNA signatures are discussed in such contexts as microarrays. The methods of the present teachings allow for defining micro RNA signatures from individual cells, such as individual stem cells and cells arising therefrom. The present teachings provide for very high levels of multiplexing, and for the derivation of signatures representing a large number of target micro RNAs from single cells.

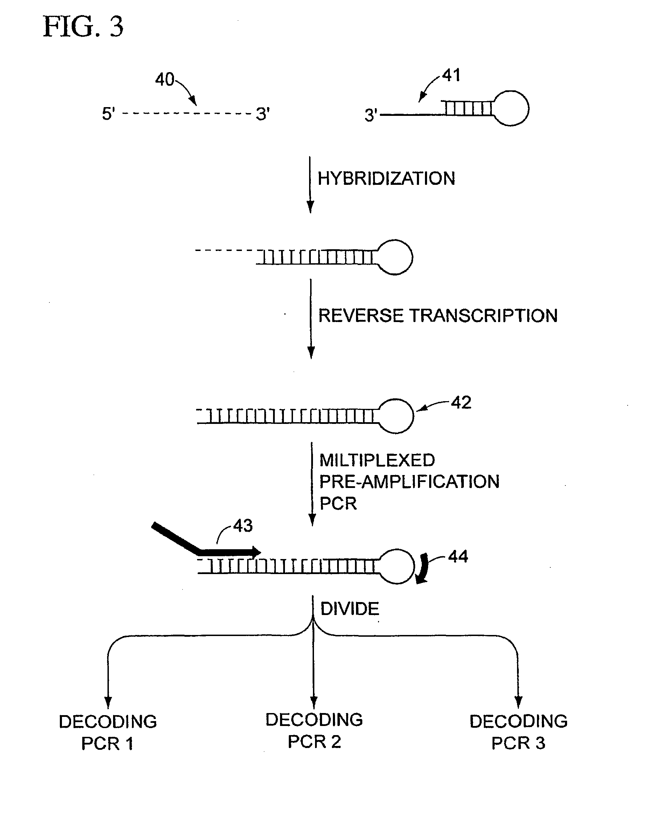

An illustrative reaction overview is depicted in Figure 3, showing a multiplex assay design according to some embodiments of the present teachings. Here, a miRNA is shown queried in a hybridization reaction comprising a stem-loop reverse transcription primer (41). Following hybridization, a reverse transcription extension reaction can be performed, optionally in a cycling procedure to form extension products (42). The extension products can then undergo a multiplexed PCR-based pre-amplification employing a micro-RNA specific forward primer (43) and a reverse primer (44). Thereafter, the products of the multiplexed PCR-based pre-amplification are divided into three separate decoding amplification reactions (here, PCR 1, PCR 2, and PCR 3).

Though not explicitly shown in Figure 3, it will be appreciated that a plurality of micro RNA species can exist in this assay, wherein a plurality of target-specific stem-loop reverse transcription primers can be employed in the reverse transcription reaction. Further, the plurality of reverse transcription reaction products can be amplified in a multiplexed PCR-based pre-amplification employing a plurality of target-specific primer pairs. Thereafter, a collection of single-plex PCR decoding reactions can be performed. For example, PCR 1 can comprise a forward primer specific for a first miRNA, PCR 2 can comprise a forward primer specific for a second miRNA, and PCR 3 can comprises a forward primer specific for a third miRNA. Each of the forward primers can further comprise a non-complementary tail portion that is uniquely zip-coded and associated with a particular micro RNA. Further, PCR 1, PCR 2, and PCR 3 can all comprise a reverse primer that was encoded by the loop of the stem-loop primers in the reverse transcription reaction. Further, PCR 1 can comprise a distinct detector probe that corresponds to the 3' end region of the first miRNA and the zip-code 1 stem of a first stem-loop reverse transcription primer, PCR 2 can comprise a distinct detector probe that corresponds to the 3' end region of the second miRNA and the zip-code 2 stem of a second stem-loop reverse transcription primer, and PCR 3 can comprise a distinct detector probe that corresponds to the 3' region of the third miRNA and the zip-code 3 stem of a third stem-loop reverse transcription primer.

Thus, in some embodiments the present teachings contemplate encoding and decoding reaction schemes, wherein an encoding multiplexed reverse transcription reaction is followed by a multiplexed encoding PCR-based pre-amplification reaction, and wherein the multiplexed encoding PCR-based pre-amplification reaction is followed by a plurality of lower-plex, for example single-plex, decoding amplification reactions.
Thus, the present teachings provide a variety of strategies to minimize the number of different molecules in multiplexed amplification reactions.

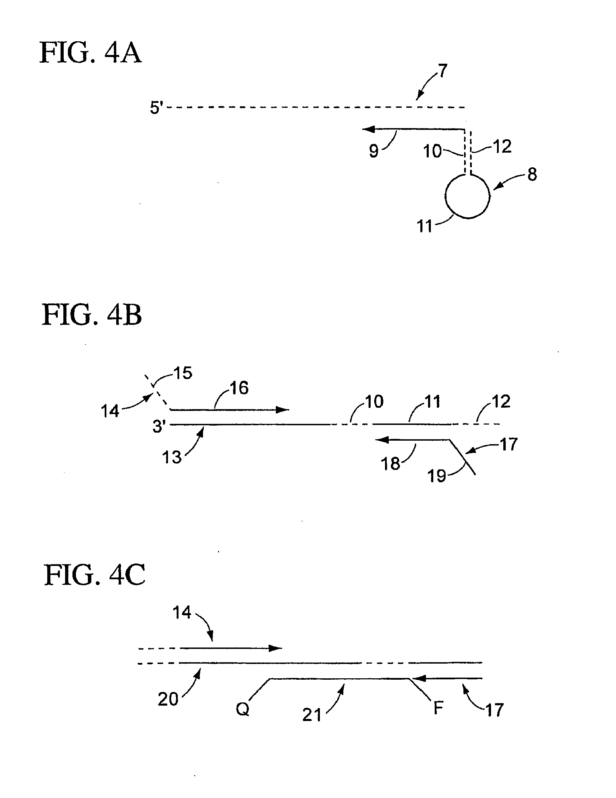

As shown in Figure 4, the present teachings provide novel methods of encoding zip-code information into an amplification product. In (A), a micro RNA sequence (7) is queried with a stem-loop reverse transcription primer (8), as can occur in a reverse transcription reaction. The architecture of the stem-loop reverse transcription primer (8) comprises a 3' target-specific portion (9), a double-stranded stem (10, 12), and a loop (11). The stem of a particular stem-loop reverse primer that queries a particular micro RNA can be zip-coded, such that a unique zip-code sequence present in the stem is associated with a particular micro RNA sequence. The 3' stem region of the stem-loop reverse transcription primer is shown as (10), and the "5' stem region of the stem-loop reverse transcription primer is shown as (12)

In Figure 2, zip-code sequence information is shown throughout as a dotted line. In (A), the stem (10, 12) comprises zip-code sequence that is distinctly associated with a particular micro RNA sequence. Extension of the 3' target-specific portion (9) of the stem-loop reverse primer (8) can result in the synthesis of a strand that is complementary to the original micro RNA target.

Following hybridization and extension of the stem-loop reverse transcription primer with the micro RNA sequence in (A), an extension reaction product results (13) in (B). This extension reaction product comprises a zip-code that was encoded by the sequence in the stem (10, 12) of the stem-loop reverse primer (8). (The zip-code encoded by the 3' stem region of the stem-loop reverse transcription primer (10) can be taken advantage of in the decoding PCR occurring in (C), as discussed below). In the PCR-based pre-amplification reaction occurring in (B), a forward primer (14) hybridizes to the 3' end of the extension reaction product (13). The forward primer (14) comprises a 3' target-specific portion (15) and a zip-code tail (16). The zip-code tail of the forward primer is a sequence that is associated with a particular micro RNA. Thus, by virtue of the first zip-code (10,12) in the stem-loop reverse primer (8) in step (A), and the second zip-code in the forward primer in step (B), a micro RNA sequence can be amplified that contains two distinct regions flanking the underlying target nucleic acid, each of which the experimentalist introduces. The PCR-based pre-amplification reaction in (B) further comprises a reverse primer (17). The reverse primer comprises a 3' target-specific portion (18) and a Tm-enhancing tail (19). Of note, the 3' target specific portion (18) of the reverse primer (17) is the sequence of the loop of the stem-loop reverse transcription primer (11) of (A).

Finally, following the PCR-based pre-amplification, an aliquot can be placed into a lower-plex decoding PCR. For example, in (C) a strand of the amplicon resulting from the PCR-based pre-amplification of (B) is shown (20). This strand can be amplified in a decoding PCR, and the original target nucleic acid quantitated. As shown in (C), the PCR can comprise a forward primer (14) and a reverse primer (17), which are the same sequences as the forward primer (14) and reverse primer (17) employed in the PCR-based pre-amplification. The decoding PCR in (C) can be analyzed in real-time, using a detector probe such as 5'-nuclease cleavable probe (21), comprising a florophore (F) and a quencher (Q). The detector probe can be highly specific for a particular amplified target nucleic acid due to the zip-code introduced in the stem-loop reverse primer in (A). The detector probe comprises sequence that is complementary to the 3' end region of the target nucleic acid, as well as the same sequence as the zip-code sequence of the 3' stem region of the stem-loop reverse transcription primer.

Applying the logic presented by the molecular architecture depicted in Figure 4, the present teachings thus provide an approach for quantifying a large number of target micro RNA sequences. For example, success has been achieved at a level of amplifying and quantitating 330 different micro RNAs, and higher levels are possible based on the present teachings. Envisioning, for example, a 1000 targets of interest, the present teachings provide a 1000-plex reverse transcription reaction, followed by a highly multiplexed PCR-based pre-amplification reaction. Aliquots from multiplexed PCR-based pre-amplification reaction can be placed in 1000 single-plex PCR decoding reactions.

A high degree of accuracy is achieved by the two zip-codes that are encoded into each amplicon. First, a zipcode is encoded into each stem-loop reverse primer by a distinct zip-code stem. Thus, for a 1000 targets of interest, 1000 different stem-loop reverse transcription primers are employed. Each of the 1000 stem-loop reverse transcription primers has a 3' target specific portion that queries a particular target of interest. Each of the 1000 stem-loop reverse primers further has a unique zip-code sequence in the stem. And, each of the 1000 stem-loop reverse primers has a loop. The loop can be the same sequence for all 1000 stem-loop reverse primers, thereby allowing a single reverse primer to be employed in the multiplexed PCR-based pre-amplification reaction. During the multiplexed PCR-based pre-amplification, 1000 different forward primers can be employed, where each of forward primer can comprise a 3' target specific portion, and a 5' zip-code tail region that contains a zip-code sequence that is uniquely associated with a particular target nucleic acid.

Finally, each of the 1000 decoding single-plex PCR can take advantage of the zip-code sequence information occurring on both ends of the amplicons resulting from the reverse transcription encoding, and the multiplexed PCR-based pre-amplification encoding. Each single-plex can comprise a primer a pair corresponding to the zip-codes employed in the reverse transcription/PCR-based pre-amplification reaction. Specifically, each single-plex decoding PCR can have a common universal reverse primer and unique forward primer. The decoding PCR can further comprise a detector probe that has sequence corresponding to the target micro RNA, as well as sequence corresponding to the zipcode stem of the stem-loop reverse primer. Such approaches can allow for a universal battery of 1000 zipcode primers to be employed in the 1000 single-plex PCRs, thus providing redundant universal primers and a corresponding reduction in cost. In some embodiments of the present teachings, each of the 1000 stem-loop reverse primers can contain the same loop. Thus, a single reverse primer sequence can be used in the PCR-based pre-amplification reaction. Further, that same reverse primer sequence can be used in the plurality of decoding PCRs. Such an approach has the benefit of minimizing the number of different molecules needed to query a large number of different targets. Of course, one need not necessarily use a single universal reverse primer. Thus, some of the stem-loop reverse primers can contain different sequences in their loops, thus resulting in the use of a corresponding set of different reverse primers in the PCR-based pre-amplifications.

An illustrative collection of sequences useful for querying the micro RNA let-7a are shown in Figure 5, and are, respectively, the forward primer, the let-7a micro RNA, the stem-loop reverse transcription primer, the detector probe, and the reverse primer.
SEQ ID NO 1: 5'GAGGTCAGGGTGAGGTAGTAGGTTGT3'
SEQ ID NO 2: 5'UGAGGUAGUAGGUUGUAUAGUU3'
SEQ ID NO 3:
   5'CATATCAACTACTCCTTTAACGGCTGAGGTGCTGTGAGGAGTAG3'
SEQ ID NO 4: 5'TTTCCTCATCAACTATAC3'
SEQ ID NO 5: 5'CTCAAGTGTCGTGGAGTCGGCAA3'

### Cycling the reverse transcription (RT) reaction

In some embodiments, the present teachings provide methods for enhanced reverse transcription primer utilization by thermal cycling. Without intending to be mechanistically limiting, the cycling methods of the present teachings are believed to disrupt errant primer low temperature associations and permit repeated homologous primer/RNA nucleations, thus increasing the amount of reverse transcription reaction product. The methods of the present teachings can be applied in a number of contexts, including increasing reverse transcription products in miRNA reverse transcription reactions, as well as increasing the reverse transcription products in multiplexed RT-PCR reactions comprising messenger RNA (mRNA) as the target nucleic acids.

Conventionally, RT reactions are performed at a single relatively low temperature, 37 - 42 °C for a long period of time (30 minutes to 1 hour, see for example Sambrook et al., 3^{rd} Edition). Typically, the aim of these reactions has been the production of long cDNA, using for example poly (T) and random primers to convert mRNA into cDNA. The long time intervals of incubation allow the RT reaction to go to completion, hopefully producing the longest cDNA molecules possible. A couple of relevant considerations provided by the present teachings include: (1) If the RNA targets are short or the defined cDNA product is short, long incubations times are not necessary for polymerase to reverse transcribe the template. (2) Single low hybridization temperatures permit non-homologous associations and intra-strand collapse to interfere with bona fide priming activity.

The kinetic principles behind homologous low temperature primer hybridization are complex and poorly defined. At these low temperatures short complementary regions can form stable intra-molecular and inter-molecular associations that interfere with bona fide complementary hybridizations. Because of the large difference in concentration between the primers and the target RNA's typically present in an RT reaction, only a small fraction of the primers anneal productively to the target sequences in a one step RT reaction. Thus, we hypothesized that conditions that (1) dissociate these primers from off target associations, (2) dissociate short intra-molecular hybridizations, and (3) permit the re-hybridization of RT primers, can allow excess un-reacted primers another chance to react with RNA target for another round of RT. Further, we hypothesized that if these conditions do not inactivate reverse transcriptase, then repetition of these conditions can increase the synthesis of cDNA until available target RNA molecules have been exhausted.

While in principle, increasing the primer concentrations from 50 - 100 nM to 1 uM or higher would be predicted to increase the number of productive primer target hybridizations, such conditions also increase problems of primer to primer interactions particularly in multiplex reactions. It was hypothesized that procedures that recycle original un-reacted primers would increase the efficiency of primer utilization and hence increase the amount of product in the RT reaction. Given that some RT enzymes can be stable up to 50 °C, 60° or higher, a temperature cycling RT reaction scheme was designed.

Thus, in some embodiments, a plurality of different target polynucleotides and a plurality of different target specific stem-loop reverse transcription primers are employed in a cycled reverse transcription reaction, followed by a multiplexed PCR-based pre-amplification reaction in the same reaction vessel.

### Cycling the RT reaction in 2 segments

In some embodiments, the first temperature (a denaturation temperature) is 47C-53C, and the second temperature (an annealing/extension temperature) is 37C-43C.

In some embodiments, the cycling reverse transcription reaction comprises at least 30 cycles of 1-5 seconds at the first temperature and 45-75 seconds at the second temperature.

In some embodiments, the cycling comprises at least 60 cycles of 1-5 seconds at the first temperature and 25-35 seconds at the second temperature.

### Cycling the RT reaction in 3 Segments

In some embodiments, the cycling comprises three segments: a low temperature segment, an intermediate temperature segment, and a high temperature segment. Without intending to be limiting, the predominating reactions occurring during each of the three segments can be considered as follows: the low temperature segment can be considered an annealing segment, the intermediate temperature segment can be considered an extension segment, and the high temperature segment can be considered a denaturation segment. Thus, in some embodiments, the cycling reverse transcription reaction can comprise an initial 16C for 30 minute incubation, followed by 60 cycles of 20C for 30 sec, 42C for 30 sec, and 50C for 1 second. These 60 cycles can be followed by a step to inactivate the reverse transcriptase, for example by elevating the temperature to 85C for 5 minutes.

In some embodiments, there can be between 50-70 cycles. In some embodiments, there can be 40-80 cycles. In some embodiments, there can be 30-100 cycles. In some embodiments, there can be greater than 100 cycles.

In some embodiments, the low temperature segment can be at 18-22C. In some embodiments, the low temperature segment can be 19-21C. In some embodiments, the low temperature segment can be 15-25C.

In some embodiments, the low temperature segment can last for 25-35 seconds. In some embodiments, the low temperature segment can last for 20-40 seconds. In some embodiments, the low temperature segment can last 15-60 seconds. In some embodiments, the low temperature segment can last longer than 60 seconds, though it will be appreciated that longer times may add unnecessary delay to the acquisition of results.

In some embodiments, the intermediate temperature segment can be 37C-45C. In some embodiments, the intermediate temperature segment can be 39C-43C.

In some embodiments, the intermediate temperature segment can last for 25-35 seconds. In some embodiments, the intermediate temperature segment can last for 20-40 seconds. In some embodiments, the intermediate temperature segment can last 15-60 seconds. In some embodiments, the intermediate temperature segment can last longer than 60 seconds, though it will be appreciated that longer times may add unnecessary delay to the acquisition of results.

In some embodiments, the high temperature segment can be 48-55C. In some embodiments, the high temperature segment can be 49-51C. In some embodiments, the high temperature segment can be higher than 55C, though it will be appreciated that higher temperatures can denature and/or destroy enzymatic activity.

In some embodiments, the high temperature segment can last 1-10 seconds. In some embodiments, the high temperature segment can last 2-8 seconds. In some embodiments, the high temperature segment can last 1-5 seconds. In some embodiments, the high temperature segment can last longer than 10 seconds, though it will be appreciated that longer times at the high temperature can denature and/or destroy enzymatic activity, especially when longer times are employed. It will further be appreciated that longer times may add unnecessary delay to the acquisition of results.

In some embodiments, especially those in which longer nucleic acids such as messenger RNAs are queried, the target-specific primer pair queries a region of the target polynucleotide that is between 100-150 nucleotides in length. As longer regions are queried, generally, incubation times can be increased, and denaturation temperatures can be increased as well.

### Kits

In certain embodiments, the present teachings also provide kits designed to expedite performing certain methods. In some embodiments, kits serve to expedite the performance of the methods of interest by assembling two or more components used in carrying out the methods. In some embodiments, kits may contain components in pre-measured unit amounts to minimize the need for measurements by end-users. In some embodiments, kits may include instructions for performing one or more methods of the present teachings. In certain embodiments, the kit components are optimized to operate in conjunction with one another.

Thus, in some embodiments the present teachings provide a kit for amplifying at least 300 short target nucleic acids, said kit comprising; at least 300 stem-loop reverse transcription primers, wherein each of the at least 300 stem-loop reverse transcription primers contains substantially the same loop sequence; at least 300 forward primers, wherein each forward primer comprises a distinct 3' target-specific portion and a distinct 5' tail; a universal reverse primer, wherein the sequence of the universal reverse primer comprises substantially the same sequence as at least 70 percent of the sequence contained in the loop of the at least 300 stem-loop reverse transcription primers, and, a Tm-enhancing tail. In some embodiments, the kit further comprises a reverse transcriptase. In some embodiments, the kit further comprises dNTPs. In some embodiments, the kit further comprises a DNA polymerase. In some embodiments, the kit further comprises a microtitre plate, wherein each of the at least 330 forward primers, and the universal reverse primer, is spotted in a separate well.

While the present teachings have been described in terms of these exemplary embodiments, the skilled artisan will readily understand that numerous variations and modifications of these exemplary embodiments are possible without undue experimentation. All such variations and modifications are within the scope of the current teachings. Aspects of the present teachings may be further understood in light of the following example, which should not be construed as limiting the scope of the teachings in any way.

### Example

Human lung and human heart RNA was purchased from Ambion Inc. Let-7a synthetic miRNA was from Integrated DNA Technologies Inc. DNA oligonucleotide primers were synthesized by Applied Biosystems.

Figures 4 and 5 depict the reaction component architecture used in this example. As shown in Figure 4, Steps A and B are multiplexed reactions with 330 sets of RT and second strand synthesis primers for almost all (330 of 332) known human miRNAs. Step B PCR amplifies the cDNA products to provide enough product for step C. Step C is done as individual singleplex TaqMan^{®} reactions in 384 well reaction plates to monitor the abundance of each of the 330 miRNAs after the multiplexed RT-PCR reactions. Note that the reverse stem-loop RT primer, forward primer (second strand synthesis and pre-PCR primer), and TaqMan^{®} probe all contain zip-coded sequences specifically assigned to each miRNA to increase the specificity of each priming reaction. In this way even small sequence differences in miRNA are amplified in subsequent reaction because any miRNA sequence difference has been amplified in PCR products and real time PCR reactions with additional specific miRNA zip-coded sequence. Also, to increase the Tm of the universal reverse primer additional sequence was added (to the 5' end of the loop sequences of the original stem-loop RT primer). This additional sequence is referred to as a Tm-enhancing tail.

### Reverse transcription

Reverse transcription reactions of 5ul contained: 0.5 ul of 10X cDNA Archiving kit buffer (Applied Biosystems), 0.335 ul MMLV reverse transcriptase (50 U/ul), 0.25 ul of 100mM dNTP, 0.065 ul of AB RNase inhibitor 20u/ul, 0.5 ul of 330 plex reverse stem-loop primer (50nM each), 2 ul of human lung purified total RNA, and 1.35 ul H20. The reaction mixture was prepared by adding 2ul of RNA sample to 3 ul of freshly prepared stock reaction mixture containing the remaining reaction ingredients for at least 10 reactions. The reaction was performed with the following incubation conditions: (20C/30s-42C/30s-50C/ls) 60 cycles. The enzyme was subsequently inactivated by incubation at 85C for 5 minutes.

### PCR-based pre-amplification

The PCR-based pre-amplification comprised 25ul, in it containing 12.5 ul of 2x Universal Mater Mix 9R) no UNG (Applied Biosystems), 5 ul of RT sample, 2.5 ul of 330 plex forward primer (500nM) each, 1.25 ul of 100uM universal reverse primer, 1.25 ul of 5u/ul AmpliTaq Gold ®, 0.5 ul of 100 mM dNTP, 0.5 ul of 100 mM MgCl2, and 1.5 ul dH20. The temperature profile for the reaction contained a 10 minute incubation at 95C to activate Taq-GOLD ®, a 55C incubation for 2 minutes, followed by 18 cycles of 95C for 1s and 65C for 1 minute.

### Real-Time PCR

The 25 ul of products from the PCR-based pre-amplification was diluted to 100ul by adding 75 ul H20. The detector probes for each TaqMan ® reaction comprised Fam on the 5 'side and quencher MGB (minor groove binder) on the 3' side. The real-time reaction mixtures contained 5ul of 2x Univeral Master Mix ® with no UNG (Applied Biosystems), 2ul of 5uM forward primer+1uM TaqMan ® probe mixture, 0.1 ul of 100 uM universal reverse primer, 0.1 ul of 4x diluted PCR-based pre-amplification sample, and 2.8 ul dH20. Real-time reaction mixtures were assembled by adding 2ul of individual forward primer+TaqMan ® probe to 8ul of freshly prepared stock solution containing the rest of the real-time PCR reagents. Real-time PCR was performed on an AB 7900 HT Sequence Detection System in a 384-well format, with the temperature regime consisting of a hot start of 95C for 10 min, followed by 40 cycles of 95C for 15s, and 60C for 1 min. The real-time PCRs for each miRNA were run in duplicate.

### Results and discussion from example

Recently (Lao et al., Biochemical and Biophysical Research Communications (2006), 343:85-89), we showed that multiple miRNAs could be profiled by partitioning the short miRNA between a short 8 nucleotide priming RT sequence that was located on the 3' end of a much longer stem loop primer and a forward, second strand synthesis primer with the rest of the miRNA sequence on its 3' end and an arbitrary Tm enhancing sequence on the 5' end. These primers were used to reverse transcribe and PCR amplify miRNA in a multiplexed manner to provide enough sample for individual singleplex real time PCR to determine the relative concentration of each of the amplified miRNAs. Although the reactions were robust over many cycles of PCR-based pre amplification, increases in the level of multiplexing added increasing scatter to plots comparing singleplex profiling with multiplex profiling of total human lung miRNA. To reduce scatter we grouped miRNA into multiplexed grouping of primers for 48 miRNAs.

The present teachings provide several new features over this, and other multiplexed approaches to micro RNA quantitation. First, the multiplicity of reverse primers and forward primers and TaqMan^{®} probes was increased to 330-plex. Second, each primer and probe was zip-coded as indicated in Figures 4 and 5. Third, extra sequences were added to the 5' end of the UR (universal reverse) primer of the pre-PCR amplification reaction to increase the Tm of this primer. Fourth, the number of pre-PCR amplification cycles was increased from 14 to 18.

Thus, Figure 4 shows one strategy according to the present teachings for dividing miRNAs into multiplexed groups. This strategy permits the multiplex assay of all miRNAs in a single group. Figure 4 shows that the primers and TaqMan^{®} probes for every miRNA are zip-coded with sequences specific to each miRNA. This means that the miRNA primers and probes for the PCR-based pre-amplification and real time PCR no longer differ from each other by the differences in actual miRNA sequence but also have large differences because of the added zip-coded sequences. The change in primer design greatly reduces primer-primer interaction from miRNA sequences containing similar or related sequence tracts. This design is anticipated to allow the multiplexed profiling of all miRNAs, including new miRNAs that are as yet undiscovered.

Results from these experiments showed that the zip-coded primer design pre-amplifies 330 miRNAs faithfully through many cycles of multiplexed PCR-based pre-amplification. The multiplexed PCR-based pre-amplifciation reactions were amplified for 1, 5, 10, 14 and 18 cycles of PCR. Then the relative concentration of each miRNA was determined by real time PCR as described in materials and methods. The theoretical differences in Ct value for each miRNA in these PCR amplifications should be 4, 5, 4, and 4 for increases in PCR cycles of from 1 to 5, from 5 to 10, from 10 to 14 and from 14 to 18 respectively. The averaged observed differences for these intervals were 4.15, 5.29, 3.87 and 4.25 respectively.

To evaluate the dynamic range of zip-coded primers, total human lung RNA was diluted from 100 ng to 10 pg and miRNA profiled in a multiplexed manner for 330 miRNAs as described in materials and methods using 18 cycles of PCR-based pre-amplification. The effect of total RNA sample size on the relative abundance of each miRNA was also analyzed, and as hypothesized a dose-response relationship found.

In these experiments 37 is the Ct value expected for single copy templates on Applied Biosystems' AB 7900 HT Sequence Detection System. However, because the miRNA was amplified with 18 cycles of PCR and then diluted 400X (a loss of 8.7 Cts), the actual Ct value of single copy DNA is 27.7 (37-18+8.7). Therefore in assessing the validity of this protocol only data points with Ct values less than 28 should be considered as meaningful. Since each of the dilution steps was a ten fold dilution, the expected difference in Ct for each miRNA for each dilution interval is 3.3. The average observed differences for miRNAs with Cts less than 28 are 2.93, 3.40, 3.86 and 3.9 for dilutions intervals from 100 ng to 10 pg respectively. The average differences from the expected are only 0.40, -0,07, -0.53 and -0.57 respectively.

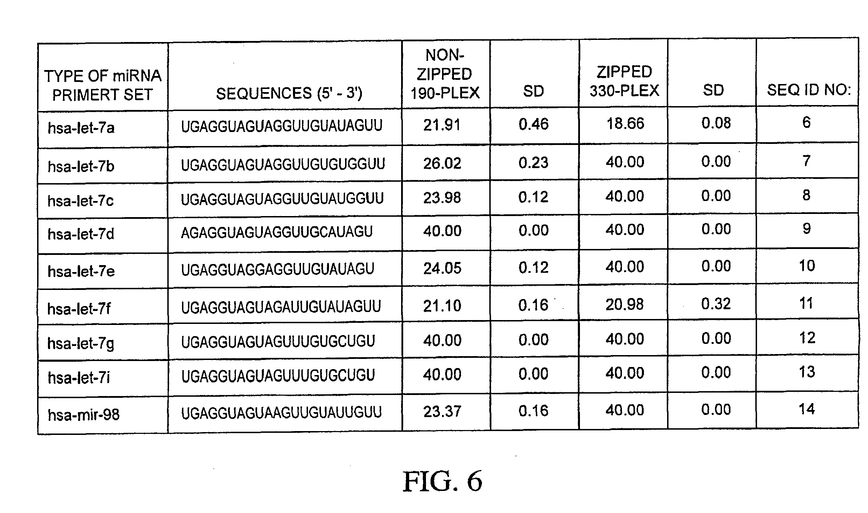

One good test for the additional specificity conferred on multiplexed RT-PCR by zip-coding the pertinent primers and probes is a direct comparison of the performance of non zip-coded primers and zip-coded primers on very closely related miRNAs. Such a group of closely related miRNA is found in the let-7 family of miRNAs. Figure 6 compares the performance of 190-plex non zip-coded primers and probes of Lao et al., Biochemical and Biophysical Research Communications (2006), 343:85-89, with the 330-plex zip-coded primers and probes of the present teachings when reacted with 100 pM of synthetic let-7a miRNA. The comparison is particularly stringent because the level of multiplexing for the zip-coded version includes 140 additional primer sets to increase the potential for spurious primer interactions. Figure 6 shows that the non zipped version of multiplexed RT-PCR has significant cross-reaction to all members of the let-7 miRNA family that have the same length as has-let-7a and only differentiates the members of the family that lack a nucleotide on the 3' end. On the other hand the zip-coded primer sets only show cross reaction to has-let-7f, which differs from has-let-7a by only one base remotely positioned from the 3' end of the forward primer. There is no cross-reaction with the other 7 members of this closely related family. The additional specificity that zip-coding confers on multiplexed RT-PCR reaction permits the miRNA profiling of all miRNAs from a single multiplexed RT-PCR reaction on total RNA samples sizes corresponding to that found in a single cell. Thus, the present teachings provide novel methods, compositions, and kits for profiling all known human miRNA in very small biopsies and single cells, including single stem cells. Further, the present teachings are flexible and robust enough to allow for increasing levels of multiplexing as new micro RNAs are discovered.

Thus, in some embodiments the present teachings provide for the multiplexed quantitation of at least 300 small nucleic acids, wherein each of the small nucleic acids is less than 30 nucleotides in length. In some embodiments, the present teachings provide for the multiplexed quantitation of at least 400 small nucleic acids, wherein each of the small nucleic acids is less than 30 nucleotides in length. In some embodiments, the present teachings provide for the multiplexed quantitation of at least 500 small nucleic acids, wherein each of the small nucleic acids is less than 30 nucleotides in length. In some embodiments, the present teachings provide for the multiplexed quantitation of at least 600 small nucleic acids, wherein each of the small nucleic acids is less than 30 nucleotides in length. In some embodiments, the present teachings provide for the multiplexed quantitation of at least 700 small nucleic acids, wherein each of the small nucleic acids is less than 30 nucleotides in length. In some embodiments, the present teachings provide for the multiplexed quantitation of at least 800 small nucleic acids, wherein each of the small nucleic acids is less than 30 nucleotides in length. In some embodiments, the present teachings provide for the multiplexed quantitation of at least 900 small nucleic acids, wherein each of the small nucleic acids is less than 30 nucleotides in length. In some embodiments, the present teachings provide for the multiplexed quantitation of at least 1000 small nucleic acids, wherein each of the small nucleic acids is less than 30 nucleotides in length. In some embodiments the dynamic range is at least 3 logs. In some embodiments the dynamic range is at least 4 logs. In some embodiments the dynamic range is at least 5 logs. In some embodiments the dynamic range is at least 6 logs. In some embodiments the dynamic range is at least 7 logs. In some embodiments the dynamic range is at least 8 logs.

Although the disclosed teachings have been described with reference to various applications, methods, kits, and compositions, it will be appreciated that various changes and modifications may be made without departing from the teachings herein. The foregoing examples are provided to better illustrate the disclosed teachings and are not intended to limit the scope of the teachings presented herein.

## Claims

1. A method of amplifying a target micro RNA comprising;
contacting the target micro RNA with a target-specific stem-loop reverse transcription primer, wherein the stem-loop reverse transcription primer comprises a 3 ' target-specific portion, a unique zip-coded stem comprising a unique zip-coded sequence that is associated with a particular micro RNA sequence, and a loop;
extending the stem-loop reverse transcription primer in a reverse transcription reaction to form a reverse transcription product;
performing a PCR-based pre-amplification on the reverse transcription product to form a PCR-based pre-amplification product, wherein the PCR-based pre-amplification comprises a forward primer and reverse primer, wherein the sequence of the reverse primer comprises substantially the same sequence as the loop of the at least one stem-loop reverse transcription primer, and a Tm-enhancing tail that is non-complementary to the reverse transcription product, and wherein the sequence of the forward primer comprises i) sequence that is complementary to the 3' end of the particular reverse transcription product sequence and ii) a 5' zip-code tail that is unique to the reverse transcription product sequence; and,
amplifying the target micro RNA.

2. The method of claim 1 for quantitating a target micro RNA,
wherein the target micro RNA is amplified by performing a decoding PCR on an aliquot of the product of the PCR-based pre-amplification, wherein the decoding PCR comprises a forward primer that is the same sequence as the forward primer in the PCR-based pre-amplification reaction, a reverse primer that comprises substantially the same sequence as the reverse primer in the PCR-based pre-amplification reaction, and, a detector probe, wherein the sequence of the detector probe comprises i) a sequence of at least 6 bases that is the same as the 3' stem region of the stem-loop reverse primer, and, ii) a sequence of at least 6 bases that is complementary to the target nucleic acid;
the method further comprising detecting the detector probe; and,
quantifying the target micro RNA.

3. The method of claim 2 for quantitating at least 300 different target micro RNAs, wherein each micro RNA is 18-30 nucleotides in length, **characterized in that**:
the at least 300 different target micro RNAs are contacted with at least 300 different target-specific stem-loop reverse transcription primers, wherein each of the at least 300 stem-loop reverse transcription primers comprises a unique 3' target-specific portion, a unique zip-coded stem comprising a unique zip-coded sequence that is associated with a particular micro RNA sequence, and a loop;
a collection of reverse transcription products is formed;
the PCR-based pre-amplification is performed on the collection of reverse transcription products to form a collection of PCR-based pre-amplification products, wherein the PCR-based pre-amplification comprises at least 300 different forward primers and at least one reverse primer, wherein the 5' zip-code tail of each the at least 300 different forward primers is unique to a particular reverse transcription product sequence;
the method further comprises dividing the collection of PCR-based pre-amplification products into at least 300 different reaction vessels;
a decoding PCR is performed in each of the at least 300 different reaction vessels;
the detector probe is detected in each of the at least 300 different reaction vessels; and,
the at least 300 different target short nucleic acids are quantified.

4. The method according to any of claims 1 to 3, wherein the reverse transcribing comprises cycling.

5. The method according to claim 4, wherein the cycling comprises 60 cycles of 20°C for 30 seconds, 42°C for 30 seconds, and 50°C for 1 second.

6. The method according to any of claims 1 to 3, wherein the PCR-based pre-amplification comprises 12-20 cycles.

7. The method according to claim 6 according to claim 3, wherein the PCR-based pre-amplification comprises 14-18 cycles.

8. The method according to claim 6 according to claim 3, wherein the 12-20 cycles each comprise 95°C for 1 second and 65°C for 1 minute.

9. The method according to claim 3, wherein the at least 300 target micro RNAs are collected from a single cell.

10. The method according to claim 3 wherein the at least 300 stem-loop reverse transcription primers comprise substantially the same loop sequence, and the sequence of the reverse primer used in the PCR-based pre-amplification reaction comprises at least 70 percent of the sequence of the loop.

11. The method according to claim 3 wherein the Tm-enhancing tail of the at least one reverse primer in the PCR-based pre-amplification reaction comprises at least 4 nucleobases.

12. The method according to claim 3 wherein the Tm-enhancing tail of the at least one reverse primer in the PCR-based pre-amplification reaction is 5 nucleobases in length.

13. The method according to claim 1 wherein the target nucleic acid is 18-30 nucleotides in length.

14. The method according to claim 1 or 2, wherein the target nucleic acid is collected from a single cell.

15. A kit for amplifying at least 300 micro RNAs, said kit comprising; at least 300 stem-loop reverse transcription primers, wherein each of the stem-loop reverse transcription primer comprises a 3' target-specific portion, a unique zip-coded stem comprising a unique zip-coded sequence that is associated with a particular micro RNA sequence, and a loop and wherein each of the at least 300 stem-loop reverse transcription primers contains substantially the same loop sequence; at least 300 forward primers, wherein each forward primer comprises a distinct 3' target- specific portion and a distinct 5' tail that is unique to the reverse transcription product sequence ; a universal reverse primer, wherein the sequence of the universal reverse primer comprises substantially the same sequence as the sequence contained in the loop of the at least 300 stem-loop reverse transcription primers, and, a Tm-enhancing tail that is non-complementary to the reverse transcription product.

16. The kit according to claim 15, further comprising a reverse transcriptase.

17. The kit according to claim 15, further comprising dNTPs.

18. The kit according to claim 15, further comprising a DNA polymerase.

19. The kit according to claim 15, further comprising a microtitre plate, wherein each of the at least 330 forward primers, and the universal reverse primer, is spotted in a separate well.

## Patentansprüche

1. Ein Verfahren zum Amplifizieren einer Ziel-micro-RNA umfassend:
Kontaktieren der Ziel-micro-RNA mit einem zielspezifischen Stamm-Schleifen-reverse-Transkriptions-Primer, wobei der Stamm-Schleifen-reverse-Transkriptions-Primer einen zielspezifischen 3'-Bereich, einen einmaligen Leitcode-markierten Stamm umfassend eine einmalige Leitcode-markierte Sequenz, die mit einer bestimmten micro-RNA-Sequenz assoziiert ist, und eine Schleife umfasst;
Verlängern des Stamm-Schleifen-reverse-Transkriptions-Primers in einer reverse-Transkriptions-Reaktion, um ein reverse-Transkriptions-Produkt zu bilden;
Durchführen einer PCR-basierten Voramplifikation an dem reverse-Transkriptions-Produkt, um ein PCR-basierte-Voramplifikations-Produkt zu bilden, wobei die PCR-basierte Voramplifikation einen Vorwärtsprimer und einen Rückwärtsprimer umfasst, wobei die Sequenz des Rückwärtsprimers im Wesentlichen dieselbe Sequenz wie die Schleife des mindestens einen Stamm-Schleifen-reverse-Transkriptions-Primers und einen Tm-erhöhenden Schwanz, der zum reverse-Transkriptions-Produkt nichtkomplementär ist, umfasst, und wobei die Sequenz des Vorwärtsprimers i) Sequenz, die zum 3'-Ende der jeweiligen reverse-Transkriptions-Produktsequenz komplementär ist, und ii) einen 5'-Leitcode-Stamm, der für die reverse-Transkriptions-Produktsequenz einmalig ist, umfasst; und
Amplifizieren der Ziel-micro-RNA.

2. Das Verfahren aus Anspruch 1 zur Quantifizierung einer Ziel-micro-RNA,
wobei die Ziel-micro-RNA amplifiziert wird mittels Durchführung einer Entschlüsselungs-PCR an einem Aliquot des Produkts der PCR-basierten Voramplifikation, wobei die Entschlüsselungs-PCR einen Vorwärtsprimer, der dieselbe Sequenz ist wie der Vorwärtsprimer in der PCR-basierten Voramplifikationsreaktion, einen Rückwärtsprimer, der im Wesentlichen dieselbe Sequenz umfasst wie der Rückwärtsprimer in der PCR-basierten Voramplifikationsreaktion, und eine Detektorsonde umfasst, wobei die Sequenz der Detektorsonde i) eine Sequenz von mindestens 6 Basen, die dieselbe ist wie die 3'-Stammregion des Stamm-Schleifen-Rückwärtsprimers, und ii) eine Sequenz von mindestens 6 Basen, die komplementär zu der Zielnukleinsäure ist, umfasst;
das Verfahren weiter umfassend Detektieren der Detektorsonde; und
Quantifizieren der Ziel-micro-RNA.

3. Das Verfahren aus Anspruch 2 zur Quantifizierung von mindestens 300 verschiedenen Ziel-micro-RNAs, wobei jede micro-RNA 18-30 Nukleotide lang ist, dadurch charakterisiert, dass:
die mindestens 300 verschiedenen Ziel-micro-RNAs mit mindestens 300 verschiedenen zielspezifischen Stamm-Schleifen-reverse-Transkriptions-Primern kontaktiert werden, wobei jeder der mindestens 300 Stamm-Schleifen-reverse-Transkriptions-Primer einen einmaligen zielspezifischen 3'-Bereich, einen einmaligen Leitcode-markierten Stamm umfassend eine einmalige Leitcode-markierte Sequenz, die mit einer bestimmten micro-RNA-Sequenz assoziiert ist, und eine Schleife umfasst;
eine Ansammlung von reverse-Transkriptions-Produkten gebildet wird;
die PCR-basierte Voramplifikation an der Ansammlung von reverse-Transkriptions-Produkten durchgeführt wird, um eine Ansammlung von PCR-basierte-Voramplifikations-Produkten zu bilden, wobei die PCR-basierte Voramplifikation mindestens 300 verschiedene Vorwärtsprimer und mindestens einen Rückwärtsprimer umfasst, wobei der 5'-Leitcode-Schwanz jeder der mindestens 300 verschiedenen Vorwärtsprimer für eine bestimmte reverse-Transkriptions-Produktsequenz einmalig ist;
das Verfahren weiter umfasst Aufteilen der Ansammlung von PCR-basierten Voramplifikationsprodukten in mindestens 300 verschiedene Reaktionsgefäße;
in jedem der mindestens 300 verschiedenen Reaktionsgefäße eine Entschlüsselungs-PCR durchgeführt wird;
die Detektorsonde in jedem der mindestens 300 verschiedenen Reaktionsgefäße detektiert wird; und
die mindestens 300 verschiedenen Ziel-kurze-Nukleinsäuren quantifiziert werden.

4. Das Verfahren gemäß irgendeinem der Ansprüche 1 bis 3, wobei das reverse Transkribieren Zyklenfahren umfasst.

5. Das Verfahren gemäß Anspruch 4, wobei das Zyklenfahren 60 Zyklen von 20°C für 30 Sekunden, 42°C für 30 Sekunden und 50°C für 1 Sekunde umfasst.

6. Das Verfahren gemäß irgendeinem der Ansprüche 1 bis 3, wobei die PCR-basierte Voramplifikation 12-20 Zyklen umfasst.

7. Das Verfahren gemäß Anspruch 6 gemäß Anspruch 3, wobei die PCR-basierte Voramplifikation 14-18 Zyklen umfasst.

8. Das Verfahren gemäß Anspruch 6 gemäß Anspruch 3, wobei die 12-20 Zyklen jeweils 95°C für 1 Sekunde und 65°C für 1 Minute umfassen.

9. Das Verfahren gemäß Anspruch 3, wobei die mindestens 300 Ziel-micro-RNAs aus einer einzigen Zelle gewonnen werden.

10. Das Verfahren gemäß Anspruch 3, wobei die mindestens 300 Stamm-Schleifen-reverse-Transkriptions-Primer im Wesentlichen dieselbe Schleifensequenz umfassen, und die Sequenz des in der PCR-basierten Voramplifikationsreaktion verwendeten Rückwärtsprimers mindestens 70 Prozent der Sequenz der Schleife umfasst.

11. Das Verfahren gemäß Anspruch 3, wobei der Tm-erhöhende Schwanz des mindestens einen Rückwärtsprimers in der PCR-basierten Voramplifikationsreaktion mindestens 4 Nukleobasen umfasst.

12. Das Verfahren gemäß Anspruch 3, wobei der Tm-erhöhende Schwanz des mindestens einen Rückwärtsprimers in der PCR-basierten Voramplifikationsreaktion 5 Nukleobasen lang ist.

13. Das Verfahren gemäß Anspruch 1, wobei die Zielnukleinsäure 18-30 Nukleotide lang ist.

14. Das Verfahren gemäß Anspruch 1 oder 2, wobei die Zielnukleinsäure aus einer einzigen Zelle gewonnen wird.

15. Ein Kit zur Amplifizierung von mindestens 300 micro-RNAs, jenes Kit umfassend:
mindestens 300 Stamm-Schleifen-reverse-Transkriptions-Primer, wobei jeder der 300 Stamm-Schleifen-reverse-Transkriptions-Primer einen zielspezifischen 3'-Bereich, einen einmaligen Leitcode-markierten Stamm umfassend eine einmalige Leitcode-markierte Sequenz, die mit einer bestimmten micro-RNA-Sequenz assoziiert ist, und eine Schleife umfasst, und wobei jeder der mindestens 300 Stamm-Schleifen-reverse-Transkriptions-Primer im Wesentlichen dieselbe Schleifensequenz enthält; mindestens 300 Vorwärtsprimer, wobei jeder Vorwärtsprimer einen individuellen zielspezifischen 3'-Bereich und einen individuellen 5'-Schwanz, der für die reverse-Transkriptions-Produktsequenz einmalig ist, umfasst; einen universellen Rückwärtsprimer, wobei die Sequenz des universellen Rückwärtsprimers im Wesentlichen dieselbe Sequenz umfasst, wie die in der Schleife der mindestens 300 Stamm-Schleifen-reverse-Transkriptions-Primer enthaltene Sequenz; und einen Tm-erhöhenden Schwanz, der zu dem reverse-Transkriptions-Produkt nichtkomplementär ist.

16. Das Kit gemäß Anspruch 15, weiter umfassend eine reverse Transkriptase.

17. Das Kit gemäß Anspruch 15, weiter umfassend dNTPs.

18. Das Kit gemäß Anspruch 15, weiter umfassend eine DNA-Polymerase.

19. Das Kit gemäß Anspruch 15, weiter umfassend eine Mikrotiterplatte, wobei jeder der der mindestens 330 Vorwärtsprimer und der universelle Rückwärtsprimer in einem separaten Loch aufgetragen ist.

## Revendications

1. Procédé d'amplification d'un micro ARN cible comprenant :
la mise en contact du micro ARN cible avec une amorce de transcription inverse de structure tige-boucle spécifique à la cible, dans lequel l'amorce de transcription inverse de structure tige-boucle comprend une partie spécifique à la cible en 3', une tige à code unique comprenant une séquence à code unique qui est associée à une séquence de micro ARN particulière, et une boucle ;
l'extension de l'amorce de transcription inverse de structure tige-boucle dans une réaction de transcription inverse pour former un produit de transcription inverse ;
la réalisation d'une pré-amplification basée sur une PCR sur le produit de transcription inverse pour former un produit de pré-amplification basée sur une PCR, dans lequel la pré-amplification basée sur une PCR comprend une amorce sens et une amorce inverse, dans lequel la séquence de l'amorce inverse comprend sensiblement la même séquence que la boucle de l'au moins une amorce de transcription inverse de structure tige-boucle, et une queue d'augmentation de la Tm qui est non complémentaire du produit de transcription inverse, et dans lequel la séquence de l'amorce sens comprend i) séquence qui est complémentaire de l'extrémité 3' de la séquence de produit de transcription inverse particulière et ii) une queue de code unique en 5' qui est propre à la séquence de produit de transcription inverse ; et
l'amplification du micro ARN cible.

2. Procédé selon la revendication 1 pour la quantification d'un micro ARN cible,
dans lequel le micro ARN cible est amplifié en réalisant une PCR de décodage sur une aliquote du produit de la pré-amplification basée sur une PCR, dans lequel la PCR de décodage comprend une amorce sens qui est la même séquence que l'amorce sens dans la réaction de pré-amplification basée sur une PCR, une amorce inverse qui comprend sensiblement la même séquence que l'amorce inverse dans la réaction de pré-amplification basée sur une PCR, et une sonde détectrice, dans lequel la séquence de la sonde détectrice comprend i) une séquence d'au moins 6 bases qui est la même que la région de la tige en 3' de l'amorce inverse de structure tige-boucle, et ii) une séquence d'au moins 6 bases qui est complémentaire de l'acide nucléique cible ;
le procédé comprenant en outre la détection de la sonde détectrice ; et,
la quantification du micro ARN cible.

3. Procédé selon la revendication 2 pour la quantification d'au moins 300 micro ARN cibles différents, dans lequel chaque micro ARN a une longueur de 18 à 30 nucléotides, **caractérisé en ce que** :
les au moins 300 micro ARN cibles différents sont mis en contact avec au moins 300 amorces de transcription inverse de structure tige-boucle spécifiques à la cible différentes, dans lequel chacune des au moins 300 amorces de transcription inverse de structure tige-boucle comprend en 3' une partie spécifique à la cible unique, une tige à code unique comprenant une séquence à code unique qui est associée à une séquence de micro ARN particulière, et une boucle ;
une collection de produits de transcription inverse est formée ;
la pré-amplification basée sur une PCR est réalisée sur la collection de produits de transcription inverse pour former une collection de produits de pré-amplification basée sur une PCR, dans lequel la pré-amplification basée sur une PCR comprend au moins 300 amorces sens différentes et au moins une amorce inverse, dans lequel la queue de code unique en 5' de chacune des au moins 300 amorces sens différentes est propre à une séquence de produit de transcription inverse particulière ;
le procédé comprend en outre la division de la collection de produits de pré-amplification basée sur une PCR dans au moins 300 récipients réactionnels différents ;
une PCR de décodage est réalisée dans chacun des au moins 300 récipients réactionnels différents ;
la sonde détectrice est détectée dans chacun des au moins 300 récipients réactionnels différents ; et
les au moins 300 acides nucléiques courts cibles différents sont quantifiés.

4. Procédé selon l'une quelconque des revendications 1 à 3, dans lequel la transcription inverse comprend un ensemble de cycles.

5. Procédé selon la revendication 4, dans lequel l'ensemble de cycles comprend 60 cycles de 20 °C pendant 30 secondes, 42 °C pendant 30 secondes, et 50 °C pendant 1 seconde.

6. Procédé selon l'une quelconque des revendications 1 à 3, dans lequel la pré-amplification basée sur une PCR comprend 12 à 20 cycles.

7. Procédé selon la revendication 6 selon la revendication 3, dans lequel la pré-amplification basée sur une PCR comprend 14 à 18 cycles.

8. Procédé selon la revendication 6 selon la revendication 3, dans lequel les 12 à 20 cycles comprennent chacun 95 °C pendant 1 seconde et 65 °C pendant 1 minute.

9. Procédé selon la revendication 3, dans lequel les au moins 300 micro ARN cibles sont collectés à partir d'une seule cellule.

10. Procédé selon la revendication 3, dans lequel les au moins 300 amorces de transcription inverse de structure tige-boucle comprennent sensiblement la même séquence de boucle, et la séquence de l'amorce inverse utilisée dans la réaction de pré-amplification basée sur une PCR comprend au moins 70 pour cent de la séquence de la boucle.

11. Procédé selon la revendication 3, dans lequel la queue d'augmentation de la Tm de l'au moins une amorce inverse dans la réaction de pré-amplification basée sur une PCR comprend au moins 4 nucléobases.

12. Procédé selon la revendication 3, dans lequel la queue d'augmentation de la Tm de l'au moins une amorce inverse dans la réaction de pré-amplification basée sur une PCR a une longueur de 5 nucléobases.

13. Procédé selon la revendication 1, dans lequel l'acide nucléique cible a une longueur de 18 à 30 nucléotides.

14. Procédé selon la revendication 1 ou 2, dans lequel l'acide nucléique cible est collecté à partir d'une seule cellule.

15. Kit pour l'amplification d'au moins 300 micro ARN, ledit kit comprenant au moins 300 amorces de transcription inverse de structure tige-boucle, dans lequel chacune des amorces de transcription inverse de structure tige-boucle comprend une partie spécifique à la cible en 3', une tige à code unique comprenant une séquence à code unique qui est associée à une séquence de micro ARN particulière, et une boucle et dans lequel chacune des au moins 300 amorces de transcription inverse de structure tige-boucle contient sensiblement la même séquence de boucle ; au moins 300 amorces sens, dans lequel chaque amorce sens comprend une partie spécifique à la cible en 3' distincte et une queue en 5' distincte qui est propre à la séquence de produit de transcription inverse ; une amorce inverse universelle, dans lequel la séquence de l'amorce inverse universelle comprend sensiblement la même séquence que la séquence contenue dans la boucle des au moins 300 amorces de transcription inverse de structure tige-boucle, et une queue d'augmentation de la Tm qui est non complémentaire du produit de transcription inverse.

16. Kit selon la revendication 15, comprenant en outre une transcriptase inverse.

17. Kit selon la revendication 15, comprenant en outre des dNTP.

18. Kit selon la revendication 15, comprenant en outre une polymérase d'ADN.

19. Kit selon la revendication 15, comprenant en outre une plaque de microtitrage, dans lequel chacune des au moins 330 amorces sens, et l'amorce inverse universelle, sont placées dans un puits séparé.
